# EUROPEAN PATENT APPLICATION

(11) **EP 3 804 802 A1**
(43) Date of publication of application: **14.04.2021**
(21) Application number: 20200881.9
(22) Date of filing: 08.10.2020
(51) Int. Cl.: A61M 39/10, A61M 39/20

(54) **PRIMING DEVICE AND SYSTEM**

(30) Priority: 10.10.2019 US 201962913344 P
(71) Applicant: EPIC MEDICAL PTE LTD, Singapore 069534 (SG)
(72) Inventor: LEE ENG HWEE, Freddie, 650621 SINGAPORE (SG)
(74) Representative: INNOV-GROUP

(57) **Abstract**

The present disclosure provides a priming device, the priming device including: a housing having a proximal end and a distal end, the housing defining an axial direction extending substantially from the proximal end to the distal end; and a membrane disposed at the proximal end, wherein the housing defines a fluid communication channel extending substantially axially from an interior surface of the membrane towards the distal end and a filter disposed at the distal end of the housing, the filter being configured to allow a passage of air. In another aspect, there is provided a priming device having an adapter.

## Description

### TECHINCAL FIELD

The present disclosure relates to the field of medical devices, and in particular to a priming device and system for removing air inside a fluid channel.

### BACKGROUND

To comply with safety standards in handling hazardous drugs such as the USP General Chapter 800 (United States Pharmacopeial Convention), hospitals and other healthcare organizations (facilities) are adopting the use of closed system transfer devices (CSTDs) in promoting exposure safe procedures that protects pharmacists, caregivers, patients and the general environment from contaminants arising from spills, aerosols and volatile organic compounds of hazardous drugs. A CSTD ensures that there is nothing from the environment enters the fluid channel containing hazardous drugs and also nothing escapes from the contents of the closed system. Hence by default, priming or the release of entrapped air from a fluid channel or body with closed system components is difficult. To facilitate this, it is common to perform priming in a laminar cabinet or chemical isolators, where the risk of microbial ingress into the devices is reduced. The pharmacist is protected with appropriate apparels and safety masks, unlike open environment in the wards. However, priming at the point of use in the ward is preferred as it controls infection better when safe and aseptic procedures are used just prior to connecting the infusion means to patients.

### SUMMARY

As will be apparent from the following, the subject matter of this disclosure enables the priming of fluid channels, like those of an intravenous (IV) line, in particular where the distal end of the infusion means or where the components used in creating the fluid communication interface with the patient are fluid and/or air tight. Novel features of this invention support the use of Closed System devices in the most vulnerable environment of a ward where nurses, caregivers, patients, and visitors would potentially be exposed to elements of hazardous drugs during connection, disconnection of an infusion means or any transfer from a fluid source container to the patient.

In one aspect, the present disclosure provides a priming device, the priming device including: a housing having a proximal end and a distal end, the housing defining an axial direction extending substantially from the proximal end to the distal end; and a membrane disposed at the proximal end, wherein the housing defines a fluid communication channel extending substantially axially from an interior surface of the membrane towards the distal end and a filter disposed at the distal end of the housing, the filter being configured to allow a passage of air. In another aspect, there is provided a priming device having an adapter.

The advantages of these and other aspects and features of various embodiments will be described with reference to the appended drawings.

### BRIEF DESCRIPTION OF DRAWINGS

Fig. 1A is a schematic view of a transfer system according to an embodiment;
Fig. 1B is a schematic view of the transfer system of Fig. 1 providing a closed fluid communication path;
Fig. 2 is a schematic view of another transfer system with the priming device;
Fig. 3 is a schematic view of the priming device according to an embodiment;
Fig. 4 is an exploded view of the priming device according to the embodiment of Fig. 3;
Fig. 5 is a cross-sectional view of the priming device according to the embodiment of Fig. 3;
Fig. 6 is a sectional view of the priming device and an adaptor according to the embodiment of Fig. 3;
Fig. 7 is a sectional view of the priming device coupled to the adaptor according to the embodiment of Fig. 6;
Fig. 8 is a sectional view of a priming device according to another embodiment;
Fig. 9 is a sectional view of a priming device according to another embodiment;
Fig. 10 is a sectional view of a priming device according to another embodiment; and
Fig. 11 is a sectional view of a priming device coupled to an adaptor according to another embodiment.

### DETAILED DESCRIPTION

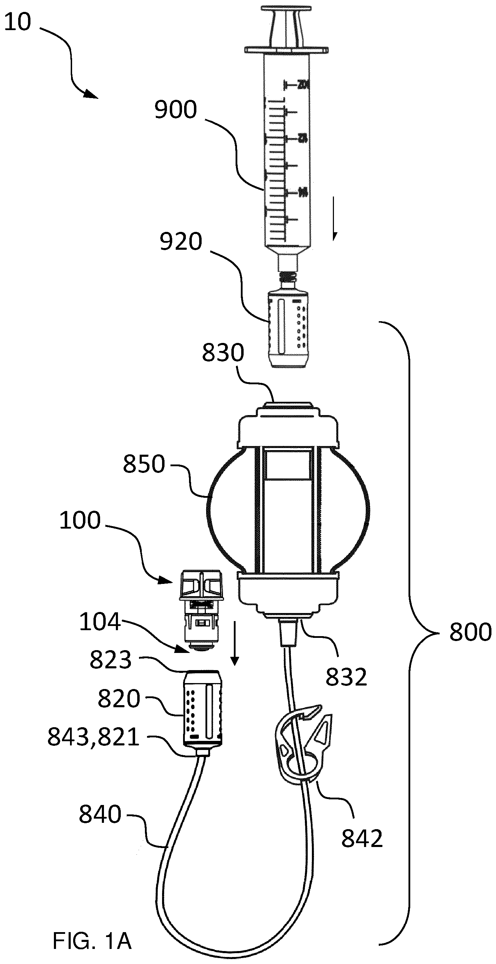

Fig. 1A illustrates an embodiment of a transfer system 800. The transfer system 800 may be configured to deliver a fluid, such as medicinal drugs to a patient through intravenous (IV) methods. The transfer system 800 may also be used in compounding drugs.

Fig. 1A shows one example in which closed system transfer devices (CSTDs) are detachably coupled provide a closed fluid system 10 suitable for facilitating safer handling of hazardous drugs. In this embodiment, the transfer system 800 may include a syringe 900. The syringe is coupled to an injector 920. The injector 920 provides a needle (not shown) within a sealed enclosure. The needle may be retractable or accessible behind a seal so that there is a mechanical barrier prohibiting the ingress of environmental contaminants into a fluid communication path extending through the needle. Additionally, traces or remnants of the fluid are not left on an exterior surface of the injector. It can thus be appreciated that the syringe 900, coupled with the injector 920, provides a sealed or closed system whereby fluid can be safely contained, stored, and/or transported

The transfer system may include a pump or any pressure source 850 having a proximal port 830 and a distal port 832. The proximal port 830 of the pump 850 may include a membrane. When the injector 920 is coupled to the pump 850, the needle of the injector 920 pierces the membrane of the proximal port 830, and thereby provides fluid communication between the syringe and the pump. The distal port 832 of the pump 850 may be coupled to a proximal end of a tubing 840. A flow regulator 842 may be provided for use with the tubing 840. A distal end 843 of the tubing 840 may be coupled to a proximal end 821 of an adaptor 820. The adaptor 820 provides a sealable closure to the tubing. The tubing 840 could also be coupled to a pumping mechanism, e.g., a peristaltic pump, to provide a pressure and/or energy source in delivering fluid through the tubing 840. In any case, it can be appreciated that the pump 850 by itself, or with the tubing 840 coupled to the adaptor 820, provides a sealed or closed system whereby fluid in the pump 830 can be safely contained, stored, and/or transported.

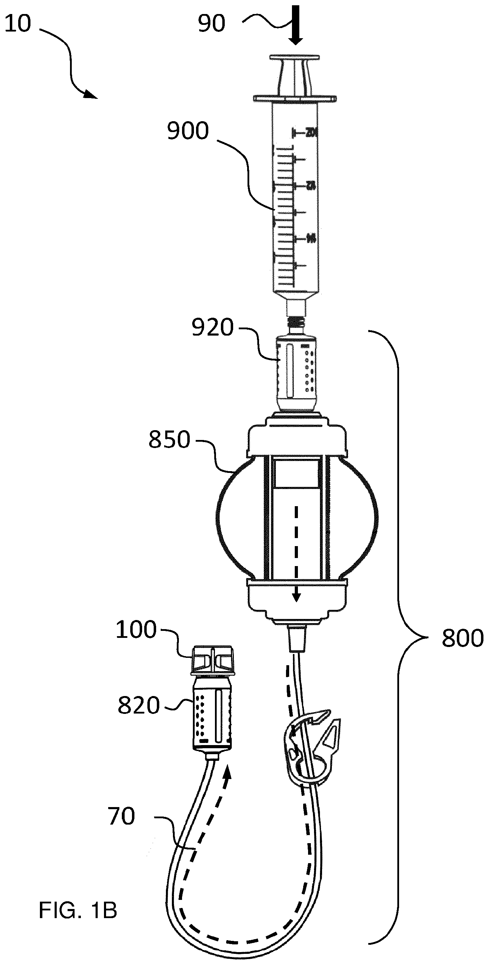

The transfer system includes a priming device 100 having a proximal end 101 which is configured so that it can be detachably coupled to a distal end 823 of the adaptor 820. Referring now to Fig. 1B, the transfer system 800 may be configured to enable closed-system priming as described in the following. In one example, fluid may be delivered from the pump 850 to the tubing 840 along a fluid communication path 70. As fluid from the pump 850 fills the tubing 840, any air previously in the tubing 840 is displaced towards the priming device 100 (partially visible in Fig. 1B). Upon completion of the priming procedure, e.g., when the tubing is completely filled with the fluid without visible air bubbles therein, the priming device 100 can be decoupled from the adaptor 820. Since the adaptor 820 provides a sealable closure for the tubing 840, the pump 850 and the tubing 840 are primed and ready for use, all the while ensuring that the fluid is mechanically prohibited from escaping outside the pump and the tubing, and that contaminants from the surroundings are also mechanically prohibited from entering the fluid communication path.

The priming device of the present disclosure can be used in a closed system (such as one shown in Fig. 1A or 1B) where the system is configured to mechanically prohibit the transfer of environmental contaminants into the system, and also to prohibit the escape of hazardous drugs or vapor concentrations outside the system. Nevertheless, the priming device of the present disclosure can also be used in a "pseudo closed" system where at least one device or one coupling in the system may not adequately provide a physical barrier to the unintended transfer of fluid out of the system or the unintended transfer of environmental contaminants into the system. In other words, the priming device of the present disclosure can be compatible for use with other devices and systems from different suppliers in the market. In some applications, the priming device of the present disclosure can be used to replace a conventional device so as to provide a closed system. In some applications, the priming device of the present disclosure can be used in place of a non-compliant or remotely compliant device (with reference to USP General Chapter 800), so that the resulting system may be deemed a closed system.

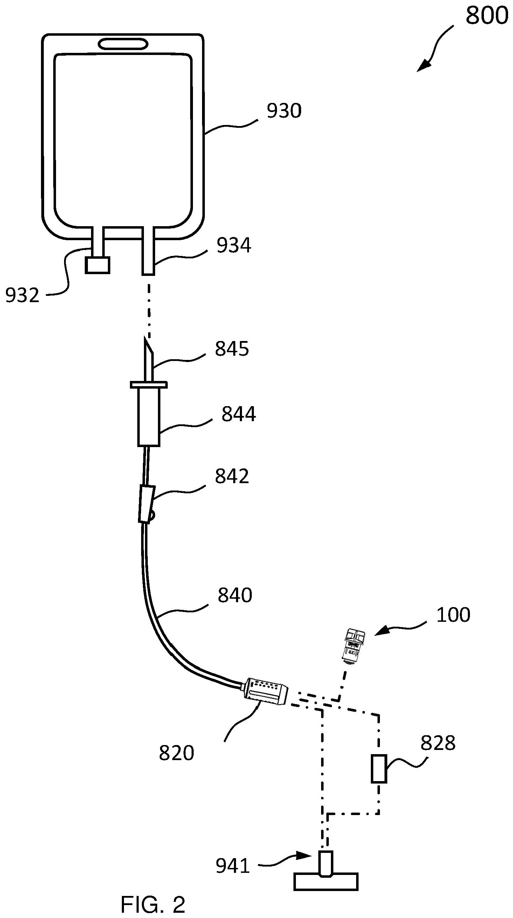

Fig. 2 schematically illustrates another transfer system 800 in which the priming device 100 may be used. An IV bag 930 is provided with a medication port 932 and a spike port 934. A drip chamber 844 may be coupled to the spike port 934 through a spike 845. Alternatively, The IV bag 930 and the tubing 840 may be coupled in another manner. The IV bag 930 and the tubing 840 may alternatively be provided to the user in a pre-assembled assembly in a sterile environment. The drip chamber 844 leads to a tubing 840, and the tubing 840 may be provided with a roller clamp 842 or another device for regulating the flow of fluid through the tubing 840. To effect priming, a distal end of the adaptor 820 may be coupled with a priming device of the present embodiment. After priming, if it is desirable to establish fluid communication with a male luer port 941, the priming device 100 may be de-coupled from the adaptor 820 without negative impact to the integrity of the transfer system. The adaptor 820 can then be safely coupled to port 941 with an appropriately designed mating interface. For example, after detaching the priming device 100 from the adaptor 820, the adaptor 820 may be coupled to a second adaptor 828 that is in turn coupled to the port 941.

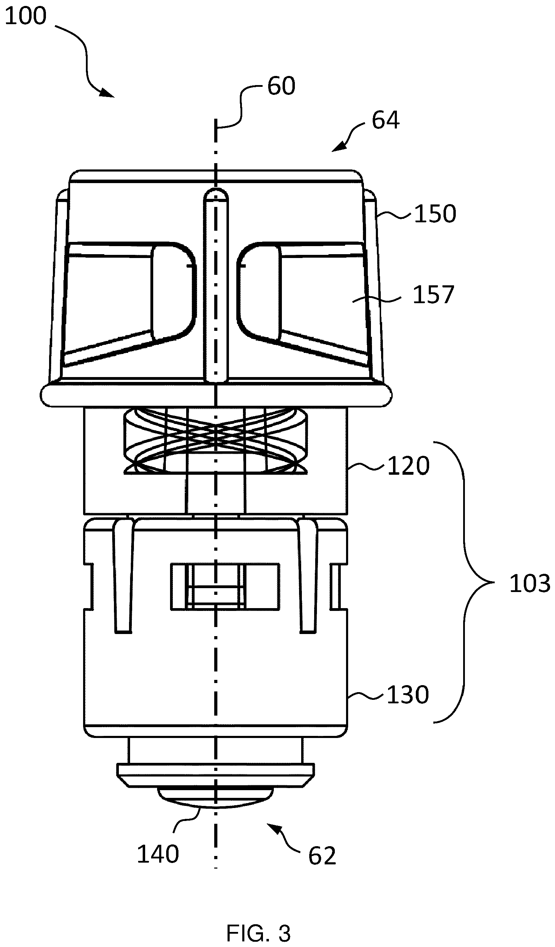

Fig. 3 illustrates an embodiment of the priming device 100. The priming device 100 defines an axis 60 extending from a proximal end 62 to a distal end 64. The priming device 100 may include a connector 103, also referred to as a dry seal connector 103. The connector 103 may include a first housing 120 coupled to a second housing 130. A distal end of the second housing 130 may be coupled to a proximal end of the first housing 120. A membrane 140 is disposed at the proximal end of the priming device 100, or at the proximal end of the second housing 130. The first housing 120 may be made of a transparent material to help the user visually determine whether there is liquid inside the priming device 100. The priming device 100 includes a third housing (retaining cap) 150 coupled to a distal end of the connector 103 or to the distal end of the first housing 120.

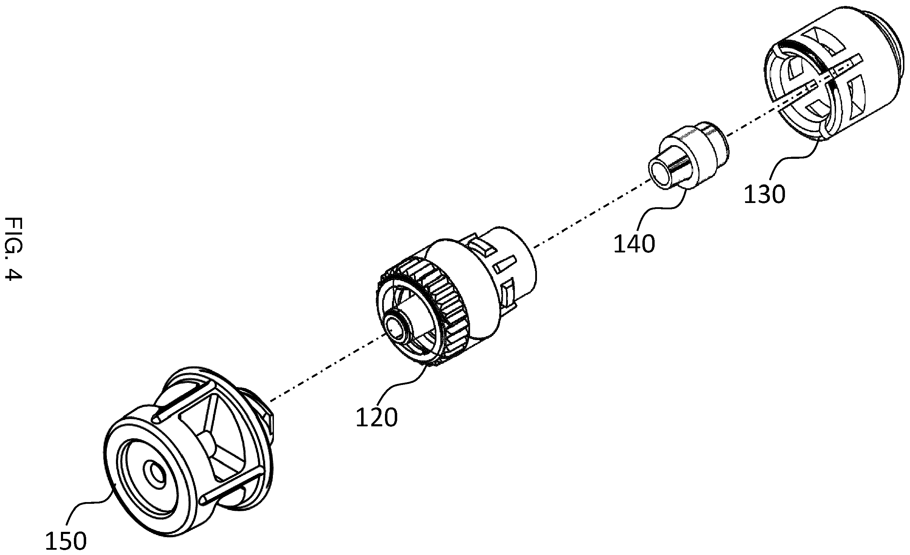

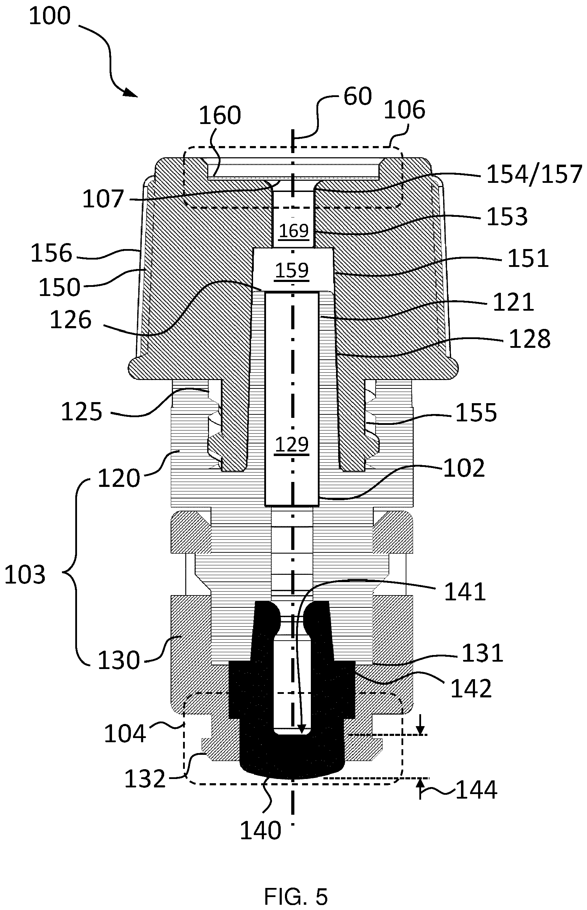

Turning next to Fig. 4 in which the priming device 100 is shown in an exploded view. The first housing 120 and the second housing 130 are configured for detachably affixed coupling by corresponding notches and tabs. In some examples, this includes the first housing 120 and the second housing 130 being configured to be manufactured as one integral or unitary piece of hardware. In some other examples, this includes the first housing 120 and the second housing 130 being configured to be manufactured as separate components, and then being assembled into an assembly, in which the assembly cannot be taken apart easily or accidentally. In some examples, the detachably affixed coupling refers to a configuration where de-coupling would render the components (such as the first housing and the second housing) incapable of being re-assembled. This helps to ensure the integrity and cleanliness standard of the product after it is shipped out by the original equipment manufacturer. The membrane 140 can be disposed between the first housing 120 and the second housing 130 so that it provides a seal throughout a mating interface 131 between the first housing 120 and the second housing 130, as shown in Fig. 5. The membrane may include a flange 142 so as to ensure that there is no leakage between the membrane and second housing. The membrane 140 may be disposed at a proximal end of the first housing 120, while extending for a length in a direction substantially parallel to the axis 60. The membrane 140 may be made of silicon or other materials capable of rendering the membrane elastically deformable.

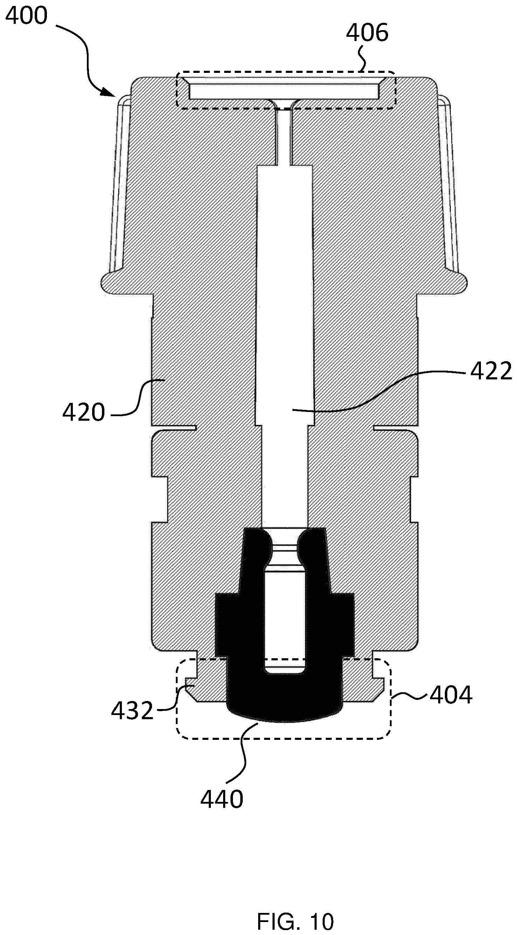

The third housing 150 may also be referred to as a retaining cap or a vent cap 150 in this document. The first housing 120 and the retaining cap 150 may be detachably coupled or be configured to be in a detachable coupling. In this document, detachable coupling includes detachably affixed coupling. For example, the proximal end of the cap 150 may be provided with a luer connect 155 that is complementary to the luer connect 125 at the distal end of the first housing 120. The luer connect 155 of the retaining cap 150 may be configured as a male luer connect, in which the luer thread is disposed on an exterior surface of the retaining cap. The luer connect 125 of the first housing 120 may be configured as a female luer connect, in which the luer thread is disposed on an interior surface of the first housing. The first housing may be further configured with a stem 128, in which the stem is configured to extend substantially axially and be received by an interior passage 159 of the retaining cap 150. In some other applications, the stem 128 and the interior passage 159 may be configured to form a clearance fit so that the retaining cap may be removed by the user. As illustrated, the stem 128 may be configured to extend a substantial length of the interior passage 159. This configuration provides a substantial interfacing area between the wall of the interior passage 159 and an exterior surface of the stem 128, and improves sealing between the retaining cap 150 and the connector 103. In some applications, the stem 128 and the interior passage 159 may be configured to form an interference fit to deter a user from detaching the retaining cap from the connector 103. In other examples of detachably affixed coupling, the retaining cap 150 and the connector 103are configured to be manufactured as an integral or unitary piece of hardware (as illustrated in Fig. 10).

The retaining cap 150 may also have an exterior surface 156 configured to provide a grip for the user. The retaining cap 150 may be configured with flanges 154 so as to define a large enough exterior diameter for a comfortable grip, while providing a small enough interior passage 159/169.

It can be seen from the cross-sectional view of Fig. 5 that a fluid communication channel 102 is defined within the priming device 100. As an example, the fluid communication channel 102 may be configured to generally extend in an orientation substantially parallel to the axis 60 (axially) of the priming device 100. The fluid communication channel 102 may be described as including a first passage 129 beginning immediately from an interior surface 141 of the membrane 140. The first passage 129 may be defined by an interior surface 121 of the first housing, together with an interior surface 141 of the membrane 140. The distal end 126 of the first housing 120 may define a step-wise increase in a transverse dimension of the fluid chamber 102 as the first passage 129 leads to a second passage 159. For example, the second passage may have a larger diameter than the first passage. References to "transverse" or "transversely" will be understood to reference an orientation or a plane substantially normal to the axis 60. The second passage 159 may be at least partially defined by an interior surface 151 of the third housing (retaining cap). There may also be a step-wise decrease in a transverse dimension of the fluid communication channel 102 as the second passage 159 leads to a third passage 169. For example, the third passage may have a smaller diameter than the second passage. The third passage may be partially defined by a distal end or a distal surface of the first housing.

Alternatively described, the fluid communication channel 102 may extend substantially axially from an interior surface 141 of the membrane 140 towards the distal end of the priming device 100. A filter 160 may be disposed at the distal end of the housing, such that the filter is configured to allow the passage of air therethrough. The fluid communication channel 102 is partially defined by the interior surface 141 of the membrane. The fluid communication channel 102 may be described as further having a fluid chamber which is configured to retain liquid. The fluid chamber 159/169 may be at least partially defined by an interior surface 121 of the housing. The fluid communication channel may extend substantially axially away from the interior surface of the membrane towards the distal end of the housing, and terminate at the fluid contact surface of the filter.

In some embodiments, the housing of the priming device can be made up of a connector 103 and a retaining cap 150. The retaining cap may be detachably coupled to the connector, for example, the retaining cap may be detachable from the connector, or the retaining cap may be undetachable from the connector. The fluid communication channel 102 may include: a first passage 129, wherein the first passage is partially defined by the interior surface of the membrane 140; and a second passage159, wherein the second passage extending substantially axially from the first passage towards the distal end of the housing, and wherein the second passage is defined by an interior surface of the connector. The second passage may be further defined by a distal end of the connector. The fluid communication channel may further include: a third passage 169 extending from the second passage, wherein the third passage is at least partially defined by an interior surface 153 of the retaining cap. The priming device may further include a filter 160 disposed at the retaining cap, and wherein the third passage terminates in the filter.

The fluid communication channel 102 is configured to receive a needle. For example, the axial dimensions of the fluid communication channel 102 are such that the fluid communication channel can accommodate a length of a needle, that is, a needle pierced through the membrane 140 will remain housed within the priming device 100.

Alternatively, the fluid communication channel 102 may extend from a proximal end of the priming device 100 generally towards a distal end of the priming device, but this does not preclude the fluid communication channel 102 from being configured with at least one bend along its length. Further the fluid communication channel 102 may comprise a circular cross section or as an alternative, the fluid communication channel 102 may comprise a non-circular cross section, or as yet another alternative, the fluid communication channel 102 may comprise an irregular cross section along its length.

Further, the priming device 100 includes a filter 160 coupled to the third housing portion 150 defining a venting portion 106 of the priming device 100. The filter 160 may be disposed at the distal end of the retaining cap, in which the filter is configured to allow the passage of air therethrough. The filter may include a hydrophobic fluid contact surface 107 which at least partially faces the fluid communication channel 102. The filter 160 may be characterized by hydrophobic properties on its fluid contact surface 107. In the embodiment of Fig. 5, the venting portion includes an aperture 157 defined by the third housing 150 at the distal end of the priming device 100. The filter 160 is disposed generally in line with the fluid communication channel 102 along the axis 60 of the priming device 100. The third housing 150 may define an indent 106 at its exterior surface, at the distal end of the priming device 100, in which the indent forms part of the venting portion 106. The indent may be further configured to position a filter 160, so that the filter 160 completely overlays the aperture. The filter 160 may be a porous hydrophobic filter that permits air to pass through, while providing a mechanical barrier to liquids. The filter 160 may thus be said to be configured to prohibit passage therethrough of molecules larger than air molecules. The filter 160 may be hydrophobic so that any liquid tends to remain in the fluid communication channel, and preferably in the second passage or in the fluid chamber 159. instead of passing through the filter 160. As a result, a liquid in the fluid communication channel 102 is prevented from exiting the priming device 100. Air in the fluid communication channel 102 may be permitted to exit the priming device 100. The filter 160 is also effective for preventing larger molecules such as vapors from the any fluid in the fluid communication channel 102 from escaping from the priming device 100.

The proximal end 104 of the priming device 100 may be configured as a dry seal connector 103 suitable to operatively couple with an injector/ adaptor 820 of a transfer system, such as an intravenous system. In some embodiments, when the proximal end 104 is coupled a complementary device such as an injector/adaptor 820, a relative sliding engagement provides a needle through the proximal end 104, resulting in a fluid communication path being established through the proximal end 104 between the injector/adaptor 820 and the priming device 100. In some other embodiments, the proximal end 104 pushes open a valve or gland of another transfer device, resulting in a fluid communication path being established between the other transfer device and the priming device 100.

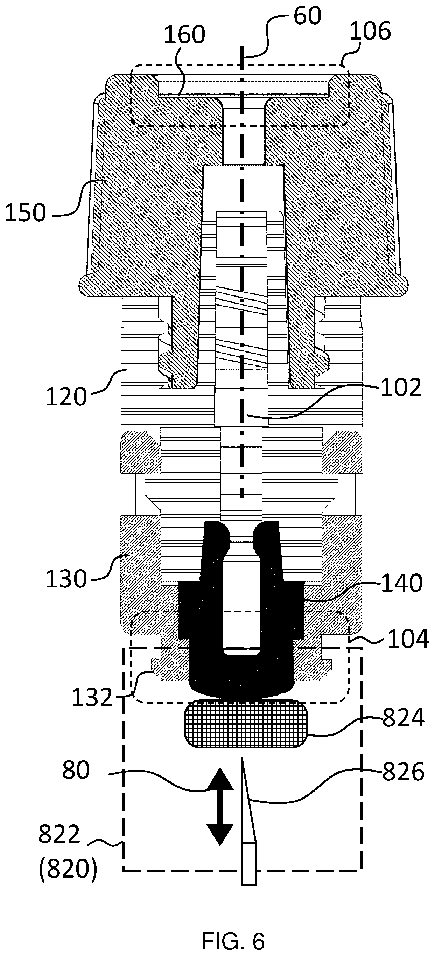

As an example, illustrated in Fig. 2, 6 and 7, an adaptor 820 of the transfer system 800 comprises a housing 822, a sealing element 824, and a needle 826. The needle 826 is configured to be movable along an engagement direction 80 substantially parallel to the axis 60 of the priming device 100. In another example, the needle 826 may move in a direction non-parallel to the axis 60 of the priming device 100. The needle 826 defines a hollow interior. The hollow interior can be in fluid communication with the tube 830 of the transfer system 800. As illustrated in Fig. 6, when the proximal end 104 of the priming device 100 couples with a corresponding adaptor 820, the membrane 140 of the priming device 100 is disposed adjacent to and abuts a sealing element 824 of the adaptor 820, forming a fluid seal therebetween. The fluid seal prohibits fluid from exiting through a coupling interface between the priming device 100 and the adaptor 820.

In an example, the adaptor 820 includes an adaptor distal end, where the proximal end of the priming device 100 and the adaptor distal end are configured to provide a detachably affixed coupling. The adaptor 820 may be detachably affixed to the priming device 100, that is, the adaptor 820 may be detachable from the priming device 100, or the adaptor 820 may be undetachable from the priming device 100. Fluid communication is established between an interior of the adaptor 820 and the fluid communication channel 102 of the priming device by the detachably affixed coupling. Alternatively, the adaptor 820 and the priming device 100 may be integrally formed.

In an embodiment, the membrane 140 is configured to be penetrable by the needle 826 creating an opening in the membrane 140, thus allowing fluid communication between the needle 826 and the fluid communication channel 102. Further, the membrane 140 is configured to elastically deform and seal the opening upon retraction of the needle 826 from the priming device 100. Additionally, the membrane 140 is configured with a membrane thickness 144 such that the membrane 140 seals the opening upon retraction of the needle 826. The membrane thickness is configured to be of a sufficient thickness to allow the elastically deformed part of the membrane to revert to its original volume and hence re-seal the opening made by the needle. The membrane thickness 144 is further configured to be of a sufficient thickness to provide a "wiping" effect on the needle as the needle is extracted out from the fluid communication channel 102, such that substantially no trace fluid remains on the surface of the needle after the extraction.

A similar arrangement may be provided between the syringe 900 and corresponding connector 830 of the transfer system 800 in achieving fluid communication between the syringe 900 and the connector 830 of the transfer system 800. Therefore, fluid communication is established between priming device 100, syringe 900 and the transfer system 800.

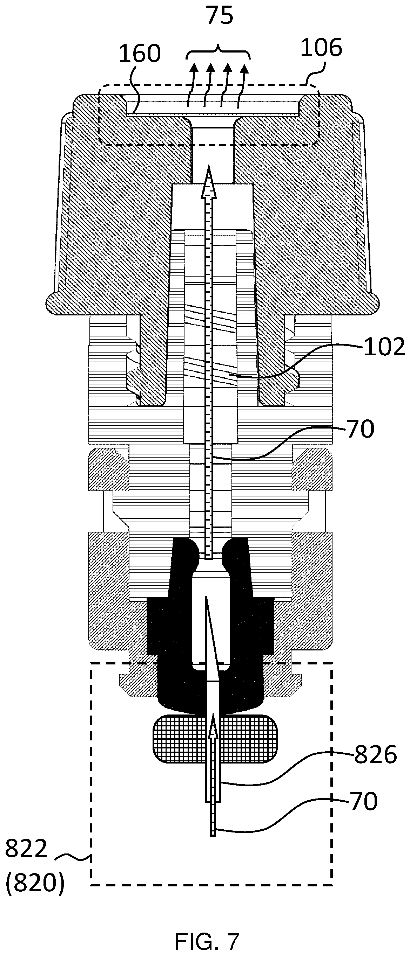

As an example, illustrated by Fig. 2 and 7, during priming, fluid 70 provided by the syringe 900 can be delivered into and through pump 830 and the tube 840 of the transfer system 800 into the priming device 100 via a pressure 90 from the syringe 900. Air within the transfer system 800 can be delivered together with the fluid 70 into the priming device 100, thereby removing the air from the transfer system 800. Alternatively, a fluid pump may be configured as periphery device for delivery of fluid 70 to the transfer system 800.

Referring to Fig. 7, the fluid 70 within the transfer system 800 is delivered into the fluid communication channel 102 of the priming device 100 via pressure 90 (Fig. 1B). In this embodiment, the liquid is sealed within the collective fluid system while the air 75 is allowed to exit the fluid system 10 through the filter 160 of the priming device 100. Therefore, air 75 is removed from the transfer system 800, and the priming device 100 may be removed for a further periphery device to be connected to adaptor 820 for delivery of fluid to a patient.

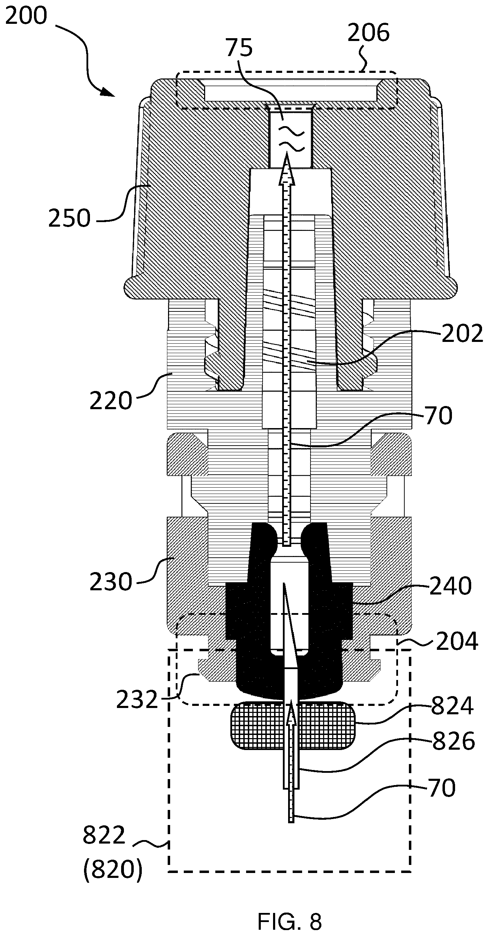

Referring to Fig. 8 for another embodiment of a priming device 200. The priming device 200 comprises a first housing portion 220, a second housing portion 230 coupled to a first end of the first housing portion 220, a third housing portion 250 coupled to a second end of the first housing portion 220. Collectively, the first housing portion 220, the second housing portion 230 and the third housing portion 250 defines a fluid communication channel 202 therethrough. Additionally, the priming device 200 includes a membrane 240 configured such that when the priming device 200 is not connected to any complementing system, fluid communication through the membrane 240 and between the fluid communication channel 202 and the atmosphere is prevented. The membrane 240 is coupled to a connecting end 232 of the second housing portion 230, such that the membrane 240 and the connecting end 232 defines part of a coupling interface 204 of the priming device 200. Further, the third housing portion 150 is sealed on one end defining a sealed portion 206 of the priming device 200.

When the coupling interface 204 of the priming device 200 is coupled with a corresponding adaptor 820, the membrane 240 of the priming device 200 is disposed adjacent to and abutting the sealing element 824 of the adaptor 820, forming a fluid seal therebetween. The fluid seal prohibits fluid from exiting through a coupling interface between the priming device 200 and the adaptor 820. Similar to above-described embodiments, the needle 826 of the adaptor 820 penetrates the sealing element 824 and subsequently the membrane 240, thereby allowing fluid communication between the adaptor 820 of the transfer system 800 and the fluid communication channel 202 of the priming device 200. During priming, fluid 70 provided by the syringe 900 is delivered into and through the transfer system 800 into the priming device 200 from the syringe 900. Air within the transfer system 800 is delivered together with the fluid 70 into the priming device 200, wherein air within the fluid communication channel 102 of the priming device 200 is trapped within the fluid communication channel 202 by the sealed portion 206. Subsequently, the air 75 is removed via decoupling the priming device 200 from the transfer system 800.

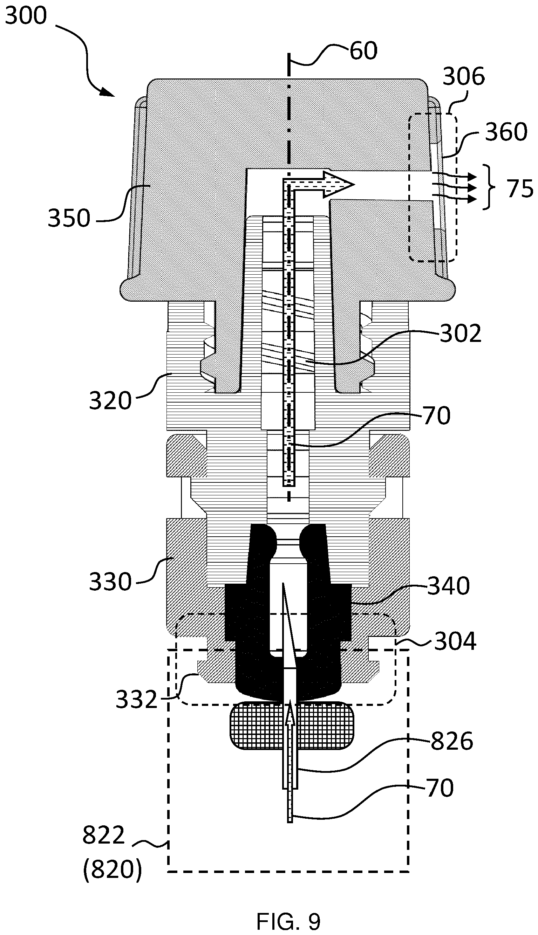

Referring to Fig. 9, illustrates another embodiment of a priming device 300. The priming device 300 comprises a first housing portion 320, a second housing portion 330 coupled to a first end of the first housing portion 320, a third housing portion 350 coupled to a second end of the first housing portion 320. Collectively, the first housing portion 320, the second housing portion 330 and the third housing portion 350 defines a fluid communication channel 302 therethrough. Additionally, the priming device 300 includes a membrane 340 configured such that when the priming device 300 is not connected to any complementing system, a fluid communication between the fluid communication channel 302 and the atmosphere through the membrane 340 is prevented. The membrane 340 is coupled to a connecting end 332 of the second housing portion 330, such that the membrane 340 and the connecting end 332 defines a coupling interface 304 of the priming device 300. Further, the priming device 300 includes a filter 360 coupled to the third housing portion 350 defining a venting portion 306 of the priming device 300. The filter 360 is disposed on a side surface of the third housing portion 350 allowing gas within the fluid communication channel 302 to exit the priming device 300 along a direction substantially transverse to the axis 60 of the priming device 300.

In another embodiment of a priming device 400, the priming device 400 comprises an integrally formed housing 420 defining a fluid communication channel 402 therethrough. Additionally, the priming device 400 includes a membrane 440 configured such that when the priming device 400 is not connected to any complementing system, a fluid communication between the fluid communication channel 402 and the atmosphere through the membrane 440 is prevented. The membrane 440 is coupled to a connecting end 432 of the housing 420, such that the membrane 440 and the connecting end 432 defines a coupling interface 404 of the priming device 400. Further, portion 406 of the housing 420 may be configured as either a venting portion or a sealed portion.

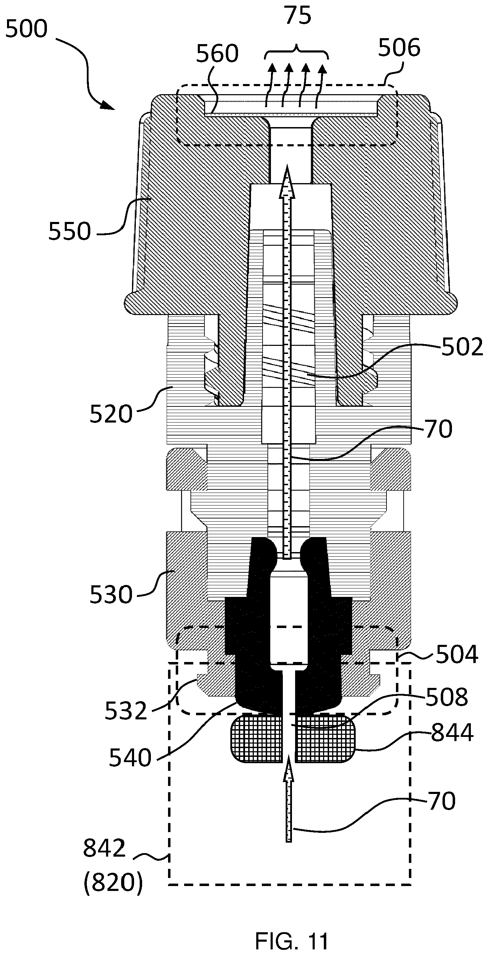

In another embodiment as illustrated in Fig. 11, a priming device 500 comprises a first housing portion 520, a second housing portion 530 coupled to a first end of the first housing portion 520, a third housing portion 550 coupled to a second end of the first housing portion 520. Collectively, the first housing portion 520, the second housing portion 530 and the third housing portion 550 defines a fluid communication channel 502 therethrough. The priming device 500 includes a membrane 540 configured such that when the priming device 500 is not connected to any complementary system, a fluid communication between the fluid communication channel 502 and the atmosphere through the membrane 540 is prevented. The membrane 540 is coupled to a connecting end 532 of the second housing portion 530, such that the membrane 540 and the connecting end 532 defines a coupling interface 504 of the priming device 50. Further, portion 506 of the third housing portion 550 may be configured as either a venting portion or a sealed portion.

The priming device 500 is coupled to an adaptor 820 of a transfer system (not shown), as illustrated in Fig. 11. The adaptor 820 includes a housing 842 and a sealing element 844. As an example, adaptor 820 is a needle safe adaptor. The adaptor 820 is configured such that when a proximal end 504 of the priming device 500 couples with the adaptor 820, deformation of the membrane 540 and the sealing element 844 causes a fluid channel 508 to be formed between the membrane 540 and the sealing element 844. The fluid channel 508 allows fluid communication between the adaptor 820 and the fluid communication channel 502. Similarly, the membrane 540 abuts sealing element 844 forming a fluid seal therebetween. The fluid seal prohibits fluid from exiting through a coupling interface between the priming device 500 and the adaptor 820. Additionally, the needle 826 is configured to pierce through both the sealing element 844 and the membrane 540 in the course of the coupling.

During priming, air within the transfer system 800 is delivered together with the fluid 70 into the priming device 500, wherein air within the fluid communication channel 502 of the priming device 500 is trapped within the fluid communication channel 502 by the sealed portion 506. Subsequently, the air can be removed through the venting portion 506 of the priming device 500. Alternatively, air can be trapped in the sealed portion 506 and removed via disconnection of the priming device 500 from the transfer system 800.

It will be readily understood that the components of the embodiments, as generally described and illustrated in the figures herein, may be arranged and designed in a wide variety of different configurations in addition to the described example embodiments. Thus, the following more detailed description of the example embodiments, as represented in conjunction with the figures, is not intended to limit the scope of the embodiments, as claimed, but is merely representative of example embodiments.

Reference throughout this specification to "one embodiment", "another embodiment" or "an embodiment" (or the like) means that a particular feature, structure, or characteristic described in connection with the embodiment is included in at least one embodiment. Thus, the appearance of the phrases "in one embodiment" or "in an embodiment" or the like in various places throughout this specification are not necessarily all referring to the same embodiment.

Furthermore, the described features, structures, or characteristics may be combined in any suitable manner in one or more embodiments. In the following description, numerous specific details are provided to give a thorough understanding of embodiments. One skilled in the relevant art will recognize, however, that the various embodiments can be practiced without one or more of the specific details, or with other methods, components, materials, etc. In other instances, some or all known structures, materials, or operations may not be shown or described in detail to avoid obfuscation.

As used herein, the singular "a" and "an" may be construed as including the plural "one or more" unless clearly indicated otherwise.

This disclosure has been presented for purposes of illustration and description but is not intended to be exhaustive or limiting. Many modifications and variations will be apparent to those of ordinary skill in the art. The example embodiments were chosen and described in order to explain principles and practical application, and to enable others of ordinary skill in the art to understand the disclosure for various embodiments with various modifications as are suited to the particular use contemplated.

Thus, although illustrative example embodiments have been described herein with reference to the accompanying figures, it is to be understood that this description is not limiting and that various other changes and modifications may be effected therein by one skilled in the art without departing from the scope or spirit of the disclosure.

## Claims

1. A priming device comprising:
a housing having a proximal end and a distal end, the housing defining an axial direction extending substantially from the proximal end to the distal end; and
a membrane disposed at the proximal end, wherein the housing defines a fluid communication channel extending substantially axially from an interior surface of the membrane towards the distal end; and
a filter disposed at the distal end of the housing, the filter being configured to allow a passage of air.

2. The priming device of claim 1, wherein the filter comprises a hydrophobic fluid contact surface, wherein the fluid contact surface is **characterized by** hydrophobic properties.

3. The priming device of claim 1, wherein the membrane is configured to be penetrable by a needle creating an opening in the membrane, allowing fluid communication between the needle and the fluid communication channel, and wherein the fluid communication channel is partially defined by the interior surface of the membrane.

4. The priming device of claim 3, wherein the fluid communication channel further comprises a fluid chamber, the fluid chamber being configured to retain liquid.

5. The priming device of claim 4, wherein the fluid communication channel extends substantially axially away from the interior surface of the membrane towards the distal end of the housing; and wherein the fluid communication channel terminates at the fluid contact surface of the filter.

6. The priming device of claim 3, wherein the housing further comprises:
a connector; and
a retaining cap, the retaining cap being detachably coupled to the connector, wherein the filter is disposed at the distal end of the retaining cap, the filter being configured to allow a passage of air.

7. The priming device of claim 6, wherein the membrane is configured to elastically deform and seal the opening upon retraction of the needle from the priming device.

8. The priming device of claim 7, wherein the membrane is configured with a membrane thickness such that the membrane seals the opening upon retraction of the needle.

9. The priming device of claim 6, wherein the fluid communication channel further comprising:
a first passage, the first passage being partially defined by the interior surface of the membrane; and
a second passage, the second passage extending substantially axially from the first passage towards the distal end of the housing, and wherein the second passage is defined by an interior surface of the connector and by a distal end of the connector.

10. The priming device of claim 9, wherein the fluid communication channel further comprises:
a third passage extending from the second passage, the third passage being at least partially defined by an interior surface of the retaining cap.

11. The priming device of claim 10, further comprising a filter disposed at the retaining cap, and wherein the third passage terminates in the filter.

12. The priming device of claim 1, further comprising:
an adaptor having an adaptor distal end, wherein the proximal end of the priming device and the adaptor distal end are configured to provide a detachably affixedcoupling, and wherein fluid communication is established between an interior of the adaptor and the fluid communication channel of the priming device by the detachably affixed coupling.

13. The priming device of claim 12, wherein the adaptor comprises a needle, the needle being disposed to pierce through the membrane in the course of the detachably affixed coupling

14. The priming device of claim 13, wherein the adaptor further comprises a sealing element, the sealing element abutting the membrane forming a fluid seal therebetween in the course of the detachably affixed coupling.

15. The priming device of claim 14, wherein the needle is configured to pierce through both the sealing element and the membrane in the course of the detachably affixed coupling.
